# EUROPEAN PATENT APPLICATION

(11) **EP 4 361 151 A1**
(43) Date of publication of application: **01.05.2024**
(21) Application number: 22827581.4
(22) Date of filing: 21.06.2022
(51) Int. Cl.: C07D 471/04, C07D 519/00, A61K 31/4375, A61P 29/00

(54) **TRICYCLIC COMPOUND USED AS GPR84 ANTAGONIST**

(30) Priority: 21.06.2021 CN 202110685568
(71) Applicant: Wuhan Humanwell Innovative Drug Research and Development Center Limited Company, Wuhan, Hubei 430075 (CN)
(72) Inventor: ZHANG, Xuejun, Wuhan, Hubei 430075 (CN); ZANG, Yang, Wuhan, Hubei 430075 (CN); WEI, Wenjun, Wuhan, Hubei 430075 (CN); LI, Qun, Wuhan, Hubei 430075 (CN); ZHAO, Xin, Wuhan, Hubei 430075 (CN); XU, Qing, Wuhan, Hubei 430075 (CN); NI, Ping, Wuhan, Hubei 430075 (CN); LI, Li'e, Wuhan, Hubei 430075 (CN); YANG, Jun, Wuhan, Hubei 430075 (CN)
(74) Representative: Haseltine Lake Kempner LLP
(86) International application number: PCT/CN2022/100224
(87) International publication number: WO 2022/268088

(57) **Abstract**

The present invention relates to a tricyclic compound used as a GPR84 antagonist, and in particular relates to a tricyclic compound having a structure shown in formula I, and tautomers, stereoisomers, hydrates, solvates, pharmaceutically acceptable salts or prodrugs thereof; the definitions of the ring Cy, L1, R1 are as described in the present invention; the tricyclic compound has significant GPR84 antagonism, good pharmaceutical developability and high safety.

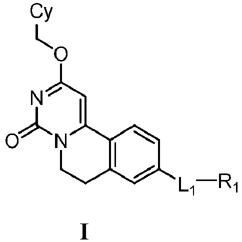

## Description

The present application claims the right of the priority of Chinese patent application 2021106855681 filed on June 21, 2021. The contents of the Chinese patent application are incorporated herein by reference in their entirety.

### TECHNICAL FIELD

The present disclosure belongs to the field of medicine, in particular to a tricyclic compound used as a GPR84 antagonist.

### BACKGROUND

G-protein-coupled receptor 84 (GPR84) is a G-protein-coupled receptor, which is coupled to the Gi/o pathway sensitive to pertussis toxin. When GPR84 is activated by binding with ligand, it can inhibit adenylate cyclase activity through Gi protein, thus reducing the level of intracellular cAMP. GPR84 belongs to fatty acid receptors, mainly expressed in bone marrow, followed by peripheral leukocytes and lungs, and can be activated by medium-chain saturated fatty acids, among which capric acid (C10), undecanoic acid (C11), and lauric acid (C12) have the best agonistic activity. In addition to endogenous ligand medium-chain fatty acids, researchers have also found some exogenous agonists with better activity, such as diindolylmethane (DIM), 6-n-octylaminouracil (6-OAU), embelin.

Under normal physiological conditions, low- and medium-chain fatty acids and diindolylmethane in immune cells can up-regulate the secretion of IL-12 P40 subunits by activating GPR84, and promote the occurrence of inflammation. Studies have shown that exogenous agonist 6-OAU causes chemotactic response by activating GPR84, upregulates AKT, ERK, and nuclear factor κB (NFκB) signaling pathways, and causes an increase in the expression levels of inflammatory mediators TNFα, IL-6, IL-12B, CCL2, CCL5, and CXCL1, and enhances the release of cytokines (IL-8, IL-12) and tumor necrosis factor α (TNF-α), thereby amplifying the inflammatory response of macrophages at the site of inflammation, aggravating the occurrence of inflammation, and triggering a variety of inflammatory diseases.

Studies have shown that higher levels of GPR84 mRNA expression are observed in fibroblasts, podocytes, proximal tubular epithelial cells, and macrophages under fibrotic conditions. In an idiopathic pulmonary fibrosis model, a GPR84 antagonist (oral, 30 mg/kg, twice daily) for 2 weeks greatly reduces the Ashcroft score from day 7. In a mouse model of endotoxemia, up-regulation of GPR84 mRNA expression has been observed during acute inflammation. In addition, up-regulation of GPR84 expression has also been observed in models of chronic inflammation such as diabetes and atherosclerosis. It has been reported that the expression of GPR84 is increased in colon tissue and blood samples of patients with inflammatory bowel disease (IBD). GPR84 mRNA transcription is also increased in liver biopsy tissues from patients with nonalcoholic fatty liver disease (NAFLD). Furthermore, the observation of GPR84 up-regulation is not limited to peripheral disease but has also been demonstrated in neuroinflammatory environment. Earlier studies reported that GPR84 was expressed at a low level in the brains of healthy adult mice, but inflammatory stimuli induced a significant up-regulation of microglia in the central nervous system (CNS), for example, in the endotoxic shock model. Tumor necrosis factor α and interleukin-1 are considered to play a key role in the up-regulation of GPR84, as the expression of GPR84 in the cerebral cortex of mice lacking these molecules is reduced. It is reported that there are also high levels of GPR84 mRNA expression in other animal models of diseases affecting the central nervous system, including experimental autoimmune encephalomyelitis, a model of multiple sclerosis, Cuprizone-induced demyelination and axotomy, and a mouse model of Alzheimer's disease (APP-PS1).

Idiopathic pulmonary fibrosis (IPF) is an unexplained, chronic, progressive fibrotic interstitial pulmonary disease characterized by progressive scarring or fibrosis confined to the interstitial spaces of the lungs, resulting in loss of lung function and eventual death. IPF carries a high risk of rapid development and death and is generally considered a rare disease. The clinical prognosis of patients with IPF is poor, with a median survival of approximately 3 years at diagnosis. Regulatory authorities have approved pirfenidone and Inetedanib for the treatment of IPF. Pirfenidone and Inetedanib can slow down the rate of decline of lung function in IPF patients. However, neither drug can improve lung function. The majority of patients continue to progress despite treatment. In addition, adverse effects of these therapies include diarrhea, abnormal liver function tests after taking Inetedanib, nausea and rash caused by pirfenidone. Therefore, there are still a large number of unmet medical needs. IPF remains a major cause of morbidity and mortality with a great need for effective treatments.

### CONTENT OF THE PRESENT INVENTION

The present disclosure aims to provide a tricyclic compound, a tautomer, a stereoisomer, a hydrate, a solvate, a pharmaceutically acceptable salt, or a prodrug thereof, which can be used for preventing and/or treating a disease related to GPR84; or for manufacturing a medicament, a pharmaceutical composition, or a preparation used as a GPR84 antagonist, or used for preventing and/or treating the disease related to GPR84.

In a first aspect of the present disclosure, provided is a tricyclic compound, a tautomer, a stereoisomer, a hydrate, a solvate, a pharmaceutically acceptable salt, or a prodrug thereof, and the tricyclic compound has a structure of formula I: 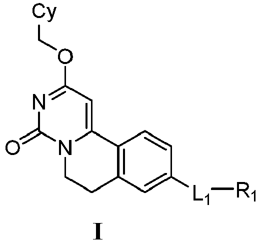 wherein
Cy is 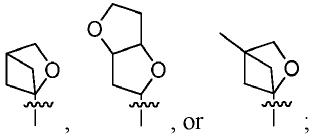
L₁ is absent or L₁ is C₁-C₄ alkylene, C₂-C₄ alkenylene with one double bond, or C₂-C₄ alkynylene with one triple bond;
R₁ is C₁-C₆ alkyl, C₁-C₆ alkoxy, 3- to 6-membered cycloalkyl, or 4- to 6-membered heterocycloalkyl;
the R₁ is optionally substituted by R₁₁;
the R₁₁ is a substituent selected from the following: halogen, cyano, hydroxyl, C₁-C₆ alkyl, C₁-C₆ alkoxy, C₃-C₆ cycloalkyl, C₁-C₆ haloalkyl, and C₁-C₆ haloalkoxy;
when there is more than one substituent, R₁₁ groups are the same or different substituents.

In a preferred embodiment of the present disclosure, L₁ is absent or L₁ is -CH₂-, - CH=CH-, or -C≡C-.

In a preferred embodiment of the present disclosure, L₁ is absent or L₁ is -C≡C-.

In a preferred embodiment of the present disclosure, R₁ is 3- to 6-membered cycloalkyl.

In a preferred embodiment of the present disclosure, R₁ is cyclopropyl, cyclobutyl, cyclopentyl, or cyclohexyl.

In a preferred embodiment of the present disclosure, R₁ is

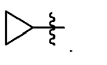

In a preferred embodiment of the present disclosure, R₁₁ is a substituent selected from the following: halogen, cyano, C₁-C₆ alkyl, and C₁-C₆ alkoxy.

In a preferred embodiment of the present disclosure, the tricyclic compound is:

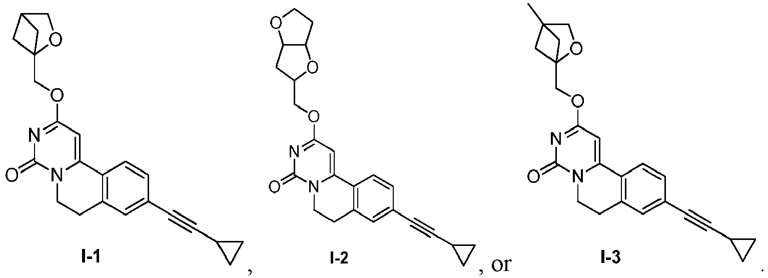

In a second aspect of the present disclosure, provided is an intermediate B having a structure of 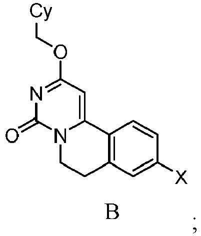
wherein Cy is as defined in the first aspect of the present disclosure;
X is selected from: -OTf, -OTs, -OMs, chlorine, bromine, or iodine.

In a third aspect of the present disclosure, provided is a preparation method for the tricyclic compound, the tautomer, the stereoisomer, the hydrate, the solvate, the pharmaceutically acceptable salt, or the prodrug thereof as described in the first aspect of the present disclosure, and the method comprises the following step: 1) reacting the intermediate B as described in the second aspect of the present disclosure with compound H-L₁-R₁ to obtain the tricyclic compound;
wherein L₁ and R₁ are as defined in the first aspect of the present disclosure.

In a preferred embodiment of the present disclosure, the method further comprises:
2) reacting the intermediate B with compound H-L₁-R₁ in the presence of a catalyst; and/or
3) reacting the intermediate B with compound H-L₁-R₁ under the protection of an inert gas; and/or
4) reacting the intermediate B with compound H-L₁-R₁ under an alkaline condition.

In a preferred embodiment of the present disclosure, the catalyst is a palladium catalyst and/or a copper catalyst.

In a preferred embodiment of the present disclosure, the palladium catalyst is selected from: Pd(PPh₃)₂Cl₂, Pd(OAc)₂, Pd(TFA)₂, PdCl₂, Pd(PPh₃)₄, and Pd₂(dba)₃; more preferably, the palladium catalyst is Pd(PPh₃)₂Cl₂ or Pd(OAc)₂.

In a preferred embodiment of the present disclosure, the copper catalyst is a monovalent copper catalyst.

In a preferred embodiment of the present disclosure, the copper catalyst is CuI.

In a preferred embodiment of the present disclosure, the inert gas is nitrogen, helium, neon, or argon.

In a preferred embodiment of the present disclosure, in the above preparation method, different reaction conditions and intermediates can be selected according to the embodiment of the present disclosure depending on the difference of each group in the compound. When there is an active group (such as carboxyl, amino, hydroxyl) in the substituent, the active group may be protected by a protecting group as required before participating in the reaction, and the protecting group is deprotected after the reaction is completed. The compounds in which one or more than one reactive site is blocked by one or more than one protecting group (also referred to as protective groups) are "protected derivatives" of the compound of formula I in the present disclosure. For example, suitable carboxyl protecting groups include benzyl, *tert-*butyl, isotopes, *etc.* Suitable amino and amido protecting groups include acetyl, trifluoroacetyl, *tert*-butoxycarbonyl, benzyloxycarbonyl, *etc.* Suitable hydroxyl protecting groups include benzyl, *etc.* Other suitable protecting groups are well known to those skilled in the art.

In a preferred embodiment of the present disclosure, in the above preparation method, the reactions in each step are preferably carried out in an inert solvent, and an appropriate inert solvent can be selected according to the specific circumstances. The inert solvent includes, but is not limited to: toluene, benzene, water, methanol, ethanol, isopropanol, ethylene glycol, N-methylpyrrolidone, dimethyl sulfoxide, tetrahydrofuran, dichloromethane, trichloromethane, 1,2-dichloroethane, acetonitrile, N,N-dimethylformamide, N,N-dimethylacetamide, dioxane, or combinations thereof.

In a fourth aspect of the present disclosure, provided is a pharmaceutical composition, and the pharmaceutical composition comprises: (a therapeutically effective amount of) the tricyclic compound, the tautomer, the stereoisomer, the hydrate, the solvate, the pharmaceutically acceptable salt, or the prodrug thereof as described in the first aspect of the present disclosure; and a pharmaceutically acceptable carrier.

In a preferred embodiment of the present disclosure, the pharmaceutical composition comprises (a therapeutically effective amount of) the tricyclic compound, the tautomer, the stereoisomer, the hydrate, the solvate, the pharmaceutically acceptable salt, or the prodrug thereof as described in the first aspect of the present disclosure; and at least one other pharmacologically active antagonist and/or inhibitor;
preferably, the other pharmacologically active antagonist is a GPR84 antagonist;
preferably, the other pharmacologically active inhibitor is a GPR84 inhibitor.

In a fifth aspect of the present disclosure, provided is a use of the tricyclic compound, the tautomer, the stereoisomer, the hydrate, the solvate, the pharmaceutically acceptable salt, or the prodrug thereof as described in the first aspect of the present disclosure, or a use of the pharmaceutical composition as described in the fourth aspect, and the use comprises:
acting as a GPR84 antagonist;
and/or, preventing and/or treating a disease related to/mediated by GPR84;
and/or, manufacturing a medicament, a pharmaceutical composition, or a preparation for acting as a GPR84 antagonist, and/or for preventing and/or treating a disease related to/mediated by GPR84.

In a preferred embodiment of the present disclosure, the disease related to GPR84 comprises: inflammatory diseases, pulmonary diseases, neuroinflammatory diseases, infectious diseases, autoimmune diseases, endocrine and/or metabolic diseases, and/or diseases related to/mediated by impaired immune function.

In a preferred embodiment of the present disclosure, the pulmonary disease is chronic obstructive pulmonary disease and/or pulmonary interstitial disease. The inflammatory disease is preferably inflammatory bowel disease or vasculitis. The pulmonary interstitial disease is preferably congenital pulmonary fibrosis.

In a preferred embodiment of the present disclosure, the pulmonary interstitial disease is preferably congenital pulmonary fibrosis or idiopathic pulmonary fibrosis.

In a preferred embodiment of the present disclosure, the autoimmune disease is rheumatoid arthritis.

The present disclosure provides a method for preventing and/or treating a disease related to GPR84 in a subject in need, and the method comprises administering to the subject an effective amount of the tricyclic compound, the tautomer, the stereoisomer, the hydrate, the solvate, the pharmaceutically acceptable salt, the prodrug thereof, or the pharmaceutical composition.

In a preferred embodiment of the present disclosure, in the method for preventing and/or treating the disease related to GPR84 in the subject in need, the disease related to GPR84 is as described above.

In a preferred embodiment of the present disclosure, all atoms in the compound have the same isotopic abundance as their natural abundance.

The additional aspects and advantages of the present disclosure will be partly given in the following description, and part of them will become apparent from the following description, or can be understood through the implementation of the present disclosure.

### Term definitions and explanations

Unless otherwise specified, the definitions of groups and terms described in the description and claims include definitions thereof as examples, exemplary definitions, preferred definitions, definitions recorded in tables, and definitions of specific compounds in the examples, *etc.*, which can be arbitrarily combined and integrated with each other. Such combined and integrated definitions of groups and compound structures should fall within the scope of the description of the present disclosure.

Unless otherwise defined, all scientific and technological terms of the present disclosure have the same meanings as those commonly understood by those skilled in the art to which the subject matter of the claims belongs. Unless otherwise specified, all patents, patent applications, and published materials cited in the present disclosure are incorporated herein by reference in their entirety. If a term has multiple definitions in the present disclosure, the definitions in this section shall prevail.

It should be understood that the above brief description and the following detailed description are exemplary and for explanatory purposes only, and do not limit the subject matter of the present disclosure in any way. In the present disclosure, the use of the singular also includes the plural unless specifically stated otherwise. It must be noted that the singular form used in the present description and claims includes the plural form of the object referred to unless clearly stated otherwise. It should also be noted that the use of "or" and "alternatively" indicates "and/or" unless otherwise specified. Furthermore, the use of the term "comprising" as well as other forms, such as "comprises", "includes", and "contains", is not limiting.

Definitions of standard chemical terms can be found in references (including Carey and Sundberg "ADVANCED ORGANIC CHEMISTRY 4THED." Vols. A (2000) and B (2001), Plenum Press, New York). Unless otherwise specified, conventional methods in the technical scope of the art, such as mass spectrometry, NMR, IR, and UV/Vis spectroscopy, and pharmacological methods are used. Unless specific definitions are provided, the terms used herein in the relevant descriptions of analytical chemistry, synthetic organic chemistry, and pharmaceuticals and medicinal chemistry are known in the art. Standard techniques can be used in chemical synthesis, chemical analysis, drug preparation, formulation, and delivery, and treatment of patients. For example, reaction and purification can be carried out according to the manufacturer's instructions for use of the kit, or in a manner known in the art or the description of the present disclosure. Generally, the above techniques and methods can be implemented according to the descriptions in a number of summary and more specific documents cited and discussed in the present disclosure according to conventional methods well-known in the art. In the present description, groups and substituents thereof can be selected by those skilled in the art to provide stable structural moieties and compounds.

When a substituent is described by a conventional chemical formula written from left to right, the substituent also includes the chemically equivalent substituent obtained when the structural formula is written from right to left. For example, CH₂O is equivalent to OCH₂. As used herein, 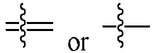 indicates the connection site of the group. As used herein, "R₁", "R1", and "R¹" have the same meaning and can be interchanged. For other symbols such as R₂, similar definitions have the same meaning.

The section headings used herein are for the purpose of organizing the article only and should not be construed as limiting the subject matter described. All documents, or portions of documents, cited in the present disclosure, including but not limited to patents, patent applications, articles, books, manuals, and treatises, are incorporated herein by reference in their entirety.

In addition to the foregoing, when used in the description and claims of the present disclosure, the following terms have the meanings shown below unless otherwise specified.

When the numerical range described in the description and claims of the present disclosure is understood as "integers", it should be understood as recording the two endpoints of the range and all integers in the range. For example, an "integer from 0 to 5" should be understood as recording every integer of 0, 1, 2, 3, 4, and 5.

In the present disclosure, the term "halogen" alone or as part of another substituent refers to fluorine, chlorine, bromine, iodine.

As used herein, the term "alkyl" alone or as part of another substituent refers to a linear or branched hydrocarbon chain group consisting only of carbon atoms and hydrogen atoms, free of unsaturated bonds, having, for example, 1 to 6 carbon atoms, and connected to the rest of the molecule by a single bond. Examples of alkyl include, but are not limited to, methyl, ethyl, *n*-propyl, isopropyl, *n*-butyl, isobutyl, *tert*-butyl, pentyl, isopentyl, neopentyl, and hexyl. Alkyl may be unsubstituted or substituted by one or more than one suitable substituent. Alkyl may also be an isotopic isomer of naturally abundant alkyl rich in isotopes of carbon and/or hydrogen (*i*.*e*., deuterium or tritium). As used herein, the term "alkenyl" refers to an unbranched or branched monovalent hydrocarbon chain that contains one or more than one carbon-carbon double bond. As used herein, the term "alkynyl" refers to an unbranched or branched monovalent hydrocarbon chain that contains one or more than one carbon-carbon triple bond.

The term "C₁-C₆ alkyl", alone or as part of another substituent, should be understood to refer to a linear or branched saturated monovalent hydrocarbon group having 1, 2, 3, 4, 5, or 6 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof. The term "C₁-C₅ alkyl" should be understood to refer to a linear or branched saturated monovalent hydrocarbon group having 1, 2, 3, or 5 carbon atoms. In particular, the group has 1, 2 or 3 carbon atoms ("C₁-C₃ alkyl"), such as methyl, ethyl, *n*-propyl, or isopropyl.

The term "C₁-C₆ alkoxy", alone or as part of another substituent, should understood as consisting of a linear or branched saturated monovalent hydrocarbon group with 1, 2, 3, 4, 5, or 6 carbon atoms and an oxygen atom, or as the definition of C₁-C₆ alkyl-O-C₁-C₆ alkyl as described herein, and the oxygen atom can be attached to any one carbon atom in the linear or linear chain of C₁-C₆ alkyl. The term "C₁-C₆ alkoxy" includes, but is not limited to: methoxy (CH₃-O-), ethoxy (C₂H₅-O-), propoxy (C₃H₇-O-), butoxy (C₄H₉-O-).

The term "halo", alone or as part of another substituent, is used interchangeably with the term "halogen-substituted". "Haloalkyl" or "halogen-substituted alkyl" refers to a branched and linear saturated aliphatic hydrocarbon group (*e*.*g*., -CvFw, where v = 1 to 3 and w = 1 to (2v + 1)) having a specific number of carbon atoms and substituted by one or more than one halogen. Examples of haloalkyl include, but are not limited to, trifluoromethyl, trichloromethyl, pentafluoroethyl, pentachloroethyl, 2,2,2-trifluoroethyl, heptafluoropropyl, and heptachloropropyl.

The term "alkenylene", alone or as part of another substituent, refers to a substituent formed by the elimination of two hydrogens from a linear or branched olefin with a specific number of carbon atoms, containing one or more than one carbon-carbon double bond and no carbon-carbon triple bonds. The carbon-carbon double bond can be located at any position in the alkenylene, and the two hydrogens eliminated can be on the same carbon atom or on different carbon atoms (for example, the two hydrogens eliminated are respectively on the carbon atoms at both ends). Thus, C₂-C₄ alkenylene includes C₂, C₃, or C₄ alkenylene; C₂ alkenylene (*i*.*e*., vinylene) includes, but is not limited to, -CH=CH-; C₃ alkenylene includes, but is not limited to, -CH₂-CH=CH-, 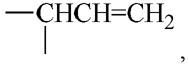 and -C(CH₃)=CH-; C₄ alkenylene includes, but is not limited to, -CH₂-CH=CH-CH₂-, 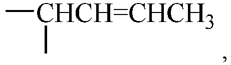 -CH₂=CH-CH₂-CH₂-, and -CH₂-CH-CH₂=CH₂-.

The term "alkynylene", alone or as part of another substituent, refers to a substituent formed by the elimination of two hydrogens from a linear or branched olefin with a specific number of carbon atoms, containing one or more than one carbon-carbon triple bond. The carbon-carbon triple bond can be located at any position in the alkynylene, and the two hydrogens eliminated can be on the same carbon atom or on different carbon atoms (for example, the two hydrogens eliminated are respectively on the carbon atoms at both ends). Thus, C₂-C₄ alkynylene includes C₂, C₃, or C₄ alkynylene; C₂ alkynylene (*i*.*e*., ethynylene) includes, but is not limited to, 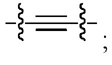 C₃ alkynylene includes, but is not limited to, 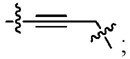 C₄ alkynylene includes, but is not limited to,

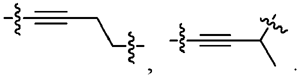

The term "cycloalkyl", alone or as part of another substituent, refers to a saturated monocyclic cyclic group consisting only of carbon atoms with a specified number of carbon atoms (*e*.*g*., C₃-C₆). Cycloalkyl includes, but is not limited to, cyclopropyl, cyclobutyl, cyclopentyl, cyclohexyl, *etc.*

The term "heterocycloalkyl", alone or as part of another substituent, refers to a cyclic group with a specified number of ring atoms (*e*.*g*., 4- to 6-membered), a specified number of heteroatoms (*e*.*g*., 1, 2, or 3) and a specified type of heteroatom (one or more of N, O, and S), which is a monocyclic ring, a bridged ring, or a spiro ring, and each ring is saturated. Heterocycloalkyl includes, but is not limited to, azetidinyl, tetrahydropyrrolyl, tetrahydrofuranyl, morpholinyl, piperidinyl, *etc.*

The term "haloalkoxy", alone or as part of another substituent, refers to alkoxy substituted by one or more than one halogen, wherein alkoxy is as defined above.

The term "inert solvent" includes, but is not limited to: toluene, benzene, water, methanol, ethanol, isopropanol, ethylene glycol, *N*-methylpyrrolidone, dimethyl sulfoxide, tetrahydrofuran, dichloromethane, trichloromethane, 1,2-dichloroethane, acetonitrile, *N,N-*dimethylformamide, *N,N*-dimethylacetamide, dioxane, or combinations thereof.

The compounds provided herein, including intermediates useful in the preparation of the compounds provided herein, contain reactive functional groups (such as, but not limited to, carboxyl, hydroxyl, and amino moieties), and also include protected derivatives thereof. "Protected derivatives" are those compounds in which one or more than one reactive site is blocked by one or more than one protecting group (also referred to as protective group). Suitable carboxyl protecting groups include benzyl, *tert*-butyl, *etc.*, as well as isotopes, *etc.* Suitable amino and amido protecting groups include acetyl, trifluoroacetyl, *tert-*butoxycarbonyl, benzyloxycarbonyl, *etc.* Suitable hydroxyl protecting groups include benzyl, *etc.* Other suitable protecting groups are well known to those skilled in the art.

In the present disclosure, "optional" or "optionally" means that the subsequently described event or circumstance may or may not occur, and the description includes both the occurrence and non-occurrence of the event or circumstance. For example, "optionally substituted aryl" means that the aryl is substituted or unsubstituted, and the description includes both substituted and unsubstituted aryl.

In the present disclosure, the term "salt" or "pharmaceutically acceptable salt" includes a pharmaceutically acceptable acid addition salt and a pharmaceutically acceptable base addition salt. The term "pharmaceutically acceptable" refers to that for those compounds, materials, compositions and/or dosage forms, they are suitable for use in contact with the tissues of human and animal without excess toxicity, irritation, allergic reactions, or other problems or complications within the scope of reliable medical judgment, and are commensurate with a reasonable benefit/risk ratio.

"Pharmaceutically acceptable acid addition salt" refers to a salt which retains the biological effectiveness of the free base without other side effects and is formed with an inorganic or organic acid. "Pharmaceutically acceptable base addition salt" refers to a salt which retains the biological effectiveness of the free acid without other side effects and is formed with an inorganic or organic base. In addition to the pharmaceutically acceptable salts, other salts may be adopted in the present disclosure. The other salts can serve as intermediates in the purification of compounds or in the preparation of other pharmaceutically acceptable salts or can be used for identifying, characterizing, or purifying the compounds of the present disclosure.

The term "stereoisomer" refers to an isomer produced by a different spatial arrangement of atoms in the molecule, including cis-trans isomers, enantiomers, diastereomers, and conformational isomers.

Depending on the selected raw materials and methods, the compounds of the present disclosure may exist in the form of one of the possible isomers or a mixture thereof, for example, as pure optical isomers, or as a mixture of isomers such as a mixture of racemic isomer and diastereoisomer, depending on the number of asymmetric carbon atoms. When describing optically active compounds, the prefixes D and L or R and S are used to denote the absolute configurations of the molecule with respect to chiral center(s) in the molecule. The prefixes D and L or (+) and (-) are symbols used to specify a rotation of plane-polarized light caused by a compound, where (-) or L indicates that the compound is levorotatory. The prefix (+) or D indicates that the compound is dextrorotatory.

When the bond with a chiral carbon in the formula of the present disclosure is depicted in a straight line, it should be understood that the two configurations (R) and (S) of the chiral carbon and both the resulting enantiomerically pure compound and mixture are included in the scope defined by the general formula. The graphical representation of the racemically or enantiomerically pure compound herein is from Maehr, J. Chem. Ed. 1985, 62:114-120. The absolute configuration of a stereocenter is represented by wedge-shaped bonds and dashed-line bonds.

The term "tautomer" refers to an isomer of a functional group resulting from a rapid movement of an atom between two positions in a molecule. The compound of the present disclosure may exhibit tautomerism. Tautomeric compounds can be present in two or more mutually convertible species. Prototropic tautomer is resulted from a migration of covalently bonded hydrogen atoms between two atoms. The tautomer generally exists in an equilibrium form, and when trying to separate a single tautomer, a mixture is usually produced, the physical and chemical properties of which are consistent with the mixture of compounds. The position of equilibrium depends on the intramolecular chemical properties. For example, for many aliphatic aldehydes and ketones, such as acetaldehyde, the ketonic form is dominant; and for phenols, the enol form is dominant. All tautomeric forms of the compounds are included in the present disclosure.

The term "pharmaceutical composition" of the present disclosure refers to a formulation of the compound of the present disclosure with a medium generally accepted in the art for delivering a biologically active compound to a mammal (*e*.*g*., a human). The medium includes a pharmaceutically acceptable carrier. The purpose of the pharmaceutical composition is to promote the administration to an organism, facilitate the absorption of the active ingredient and thus exert its biological activity.

In the present disclosure, "pharmaceutically acceptable carrier" includes, but is not limited to, any acceptable adjuvants, carriers, excipients, glidants, sweeteners, diluents, preservatives, dyes/coloring agents, flavoring agents, surfactants, wetting agents, dispersants, suspending agents, stabilizers, isotonic agents, solvents, or emulsifiers for humans or livestocks as licensed by relevant governmental administrations.

In the present disclosure, the term "solvate" refers to the compound of the present disclosure or a salt thereof including a stoichiometric or non-stoichiometric solvent bonded through an intermolecular non-covalent force. When the solvent is water, the solvate is a hydrate.

In the present disclosure, the term "prodrug" can be converted into the compound of the present disclosure having biological activity under physiological conditions or through solvents. The prodrug of the present disclosure is prepared by modifying the functional groups in the compound, and the modification can be removed by conventional operations or *in vivo*, so as to obtain the parent compound. The prodrug includes a compound formed by attaching a hydroxyl or amino group in the compound of the present disclosure to any group. When the prodrug of the compound of the present disclosure is administered to a mammal individual, the prodrug is dissociated to form a free hydroxyl group and a free amino group respectively.

The compound of the present disclosure may contain an unnatural proportion of atomic isotope at one or more than one atom(s) that constitute the compound. For example, the compound can be labeled with a radioactive isotope, such as deuterium (²H), tritium (³H), iodine -125(¹²⁵I), or C-14 (¹⁴C). All isotopic variations of the compound of the present disclosure, whether radioactive or not, are encompassed within the scope of the present disclosure.

In the present disclosure, the term "excipient" refers to a pharmaceutically acceptable inert ingredient. Examples of categories of the term "excipient" include, but are not limited to, binders, disintegrants, lubricants, glidants, stabilizers, fillers, diluents, *etc.* Excipients can enhance operation properties of the pharmaceutical formulation, *i*.*e*., allowing the formulation to be more suitable for direct compression by increasing fluidity and/or adhesion.

As used herein, the term "treatment" and other similar synonyms include the following meanings:
(i) preventing the occurrence of a disease or condition in mammals, particularly when such mammals are susceptible to the disease or condition but have not been diagnosed as having the disease or condition;
(ii) inhibiting the disease or condition, *i*.*e*., restraining its development;
(iii) ameliorating the disease or condition, *i*.*e*., causing the disease or condition to subside; or
(iv) alleviating the symptoms caused by the disease or condition.

For the reaction of each step, the reaction temperature can be appropriately selected according to the solvent, starting material, reagent, *etc.*, and the reaction time can also be appropriately selected according to the reaction temperature, solvent, starting material, reagent, *etc.* After the reaction of each step, the target compound can be separated and purified from the reaction system by common methods, such as filtration, extraction, recrystallization, washing, silica gel column chromatography and other methods. Without affecting the next reaction step, the target compound can also be directly used in the next reaction step without separation and purification. The reaction of each step of the present disclosure is preferably carried out in an inert solvent, and the inert solvent includes, but is not limited to: toluene, benzene, water, methanol, ethanol, isopropanol, ethylene glycol, N-methylpyrrolidone, dimethyl sulfoxide, tetrahydrofuran, dichloromethane, trichloromethane, 1,2-dichloroethane, acetonitrile, N,N-dimethylformamide, N,N-dimethylacetamide, dioxane, or combinations thereof.

### Beneficial effects

The present inventors have unexpectedly developed a tricyclic compound, a tautomer, a stereoisomer, a hydrate, a solvate, a pharmaceutically acceptable salt, or a prodrug thereof used as a GPR84 antagonist after extensive and intensive research, and the tricyclic compound has strong GPR84 antagonistic effects. The compound of the present disclosure has high oral exposure in mice, exhibits excellent pharmacokinetic properties, and has a high free fraction in human plasma, good stability, slow metabolism, good druggability; low risk of drug interaction, and good safety.

The compound of the present disclosure can be used as a GPR84 antagonist to prevent and/or treat a disease related to GPR84; for the manufacture of a medicament, a pharmaceutical composition, or a preparation used as the GPR84 antagonist, and for the manufacture of a medicament, a pharmaceutical composition, or a preparation used for preventing and/or treating the disease related to GPR84.

### DETAILED DESCRIPTION OF THE PREFERRED EMBODIMENT

The present disclosure is further illustrated below in conjunction with specific examples. It should be understood that the following description is only the most preferred embodiment of the present disclosure and should not be construed as limiting the scope of protection of the present disclosure. On the basis of a full understanding of the present disclosure, the experimental methods without indication of specific conditions in the following examples shall be implemented usually in accordance with conventional conditions or the conditions suggested by the manufacturer. Those skilled in the art can make non-essential modifications to the technical solutions of the present disclosure, and such modifications should be considered to be included in the scope of protection of the present disclosure.

In the following examples, DCM stands for dichloromethane; MeOH stands for methanol; TEA stands for triethylamine; DMF stands for N,N-dimethylformamide; EA stands for ethyl acetate.

### Example 1: Preparation of compound I-1

The synthetic route is as follows:

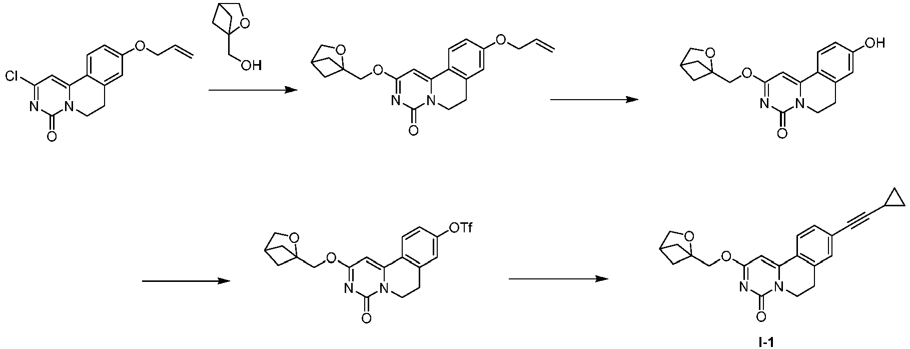

### Step 1: Synthesis of 2-((2-oxabicyclo[2.1.1]hexan-1-yl)methoxy)-9-(allyloxy)-6,7-dihydro-4H-pyrimido[6,1-α]isoquinolin-4-one

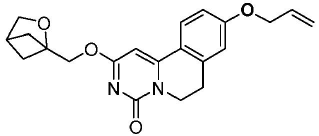

(2-Oxabicyclo[2.1.1]hexan-1-yl)methanol (0.23 g, 2.1 mmol) was dissolved in a solution of anhydrous DCM (100 mL) under nitrogen atmosphere at 0°C, then sodium hydride (0.081 g, 2.1 mmol, content of 60% in mineral oil) was added thereto, and the mixture was stirred for 15 minutes. 9-(Allyloxy)-2-chloro-6,7-dihydro-4H-pyrimido[6,1-α]isoquinolin-4-one (0.5 g, 1.6 mmol) was added thereto, and the reaction mixture was warmed to room temperature and stirred overnight. Saturated NH₄Cl aqueous solution (50 mL) was added thereto to wash once. The organic layer was washed once with water (50 mL), dried over MgSO₄, and concentrated under reduced pressure to obtain the crude product, and the crude product was purified by a silica gel column to obtain the product 2-((2-oxabicyclo[2.1.1]hexan-1-yl)methoxy)-9-(allyloxy)-6,7-dihydro-4H-pyrimido[6,1-α]isoquinolin-4-one (0.45 g, yield of 79%), LC-MS, M/Z (ESI): 367.2 [M+H]⁺.

### Step 2: Synthesis of 2-((2-oxabicyclo[2.1.1]hexan-1-yl)methoxy)-9-hydroxy-6,7-dihydro-4H-pyrimido[6,1-α]isoquinolin-4-one

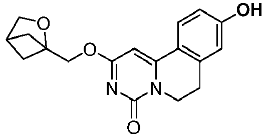

Compound 2-((2-oxabicyclo[2.1.1]hexan-1-yl)methoxy)-9-(allyloxy)-6,7-dihydro-4H-pyrimido[6,1-α]isoquinolin-4-one (0.45 g, 1.23 mmol) was placed in a 100 mL single-necked flask, then DCM/MeOH (10 mL/10 mL) was added thereto, and K₂CO₃ (0.34 g, 2.46 mmol) and Pd(PPh₃)₄ (0.071 g, 0.061 mmol) were added thereto at room temperature. The reaction mixture was stirred overnight at room temperature. The reaction mixture was detected by thin-layer chromatography. After the reaction was completed, the reaction solvent was concentrated to dryness to obtain the crude product, and the crude product was diluted with EA (20 mL). The organic phase was extracted three times with water (20 mL × 3), and the aqueous phases were combined, and then the pH of the aqueous phases was adjusted to 4. The aqueous phase was then extracted three times with DCM (20 mL × 3), and the organic phases were combined, washed once with saturated brine (20 mL × 1), then dried, filtered, and concentrated to obtain the crude product 2-((2-oxabicyclo[2.1.1]hexan-1-yl)methoxy)-9-hydroxy-6,7-dihydro-4H-pyrimido[6,1-α]isoquinolin-4-one(0.35 g, yield of 87%), LC-MS, M/Z (ESI): 327.3 [M+H]⁺.

### Step 3: Synthesis of 2-((2-oxabicyclo[2.1.1]hexan-1-yl)methoxy)-4-oxo-6,7-dihydro-4H-pyrimido[6,1-α]isoquinolin-9-yl trifluoromethanesulfonate

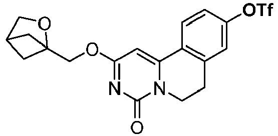

Compound 2-((2-oxabicyclo[2.1.1]hexan-1-yl)methoxy)-9-hydroxy-6,7-dihydro-4H-pyrimido[6,1-α]isoquinolin-4-one (0.35 g, 1.07 mmol) was dissolved in DCM (10 mL). 1,1,1-Trifluoro-N-phenyl-N-((trifluoromethyl)sulfonyl)methanesulfonamide (0.46 g, 1.28 mmol) and TEA (0.27 mL, 1.93 mmol) were added thereto, then the reaction mixture was stirred at room temperature for 5 hours, and the solvent was concentrated to dryness to obtain the crude product which was purified by a silica gel column (DCM: MeOH (V/V) = 50:1 to 20:1) to obtain 2-((2-oxabicyclo[2.1.1]hexan-1-yl)methoxy)-4-oxo-6,7-dihydro-4H-pyrimido[6,1-α]isoquinolin-9-yl trifluoromethanesulfonate (0.31 g, yield of 63.4%), LC-MS, M/Z (ESI): 459.1 [M+H]⁺.

### Step 4: Synthesis of 2-((2-oxabicyclo[2.1.1]hexan-1-yl)methoxy)-9-(cyclopropylethynyl)-6,7-dihydro-4H-pyrimido[6,1-α]isoquinolin-4-one

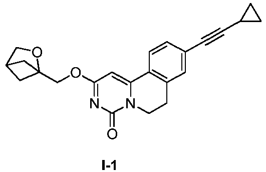

Pd(PPh₃)₂Cl₂ (0.011 g, 0.016 mmol), CuI (0.012 g, 0.065 mmol), and 2-((2-oxabicyclo[2.1.1]hexan-1-yl)methoxy)-4-oxo-6,7-dihydro-4H-pyrimido[6,1-α]isoquinolin-9-yl trifluoromethanesulfonate (0.15 g, 0.33 mmol) were placed in a sealed tube under nitrogen atmosphere. Cyclopropylacetylene (0.11 g, 1.64 mmol) and triethylamine (0.17 g, 1.64 mmol) were dissolved in anhydrous DMF (3 mL), added to the sealed tube and stirred overnight at 60°C. The reaction mixture was detected by thin-layer chromatography. After the raw materials were completely reacted, the reaction mixture was concentrated to remove DMF to obtain the crude product, which was purified by a silica gel column (dichloromethane: methanol (V/V) = 50:1 to 10:1) to obtain 2-((2-oxabicyclo[2.1.1]hexan-1-yl)methoxy)-9-(cyclopropylethynyl)-6,7-dihydro-4H-pyrimido[6,1-α]isoquinolin-4-one (I-1) (63.6 mg, yield of 51.9%).

LC-MS, M/Z (ESI): 375.2 [M+H]⁺.

¹H NMR (400 MHz, CDCl₃) δ 7.58 (d, J = 8.2 Hz, 1H), 7.34 (dd, J = 8.2, 1.5 Hz, 1H), 7.28 (d, J = 1.0 Hz,1H), 6.37 (s, 1H), 4.69 (s, 2H), 4.24 - 4.11 (m, 2H), 3.84 (d, J = 8.8 Hz, 2H), 2.95 (dd, J = 7.2, 4.8 Hz,3H), 1.84 (dt, J = 4.8, 4.3 Hz, 2H), 1.59 (dd, J = 4.7, 1.8 Hz, 2H), 1.47 (tt, J = 8.2, 5.1 Hz, 1H), 0.96 -0.88 (m, 2H), 0.87 - 0.80 (m, 2H).

### Example 2: Preparation of target compound I-2

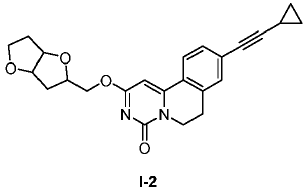

The synthetic route of compound I-2 referred to the synthetic method of I-1, and (2-oxabicyclo[2.1.1]hexan-1-yl)methanol was replaced by (hexahydrofuro[3,2-b]furan-2-yl)methanol. After a similar four-step reaction, 9-(cyclopropylethynyl)-2-((hexahydrofuro[3,2-b]furan-2-yl)methoxy)-6,7-dihydro-4H-pyrimido[6,1-α]isoquinolin-4-one (I-2) was obtained. LC-MS, M/Z (ESI): 405.2 [M+H]⁺.

### Example 3: Preparation of target compound I-3

The synthetic route is as follows:

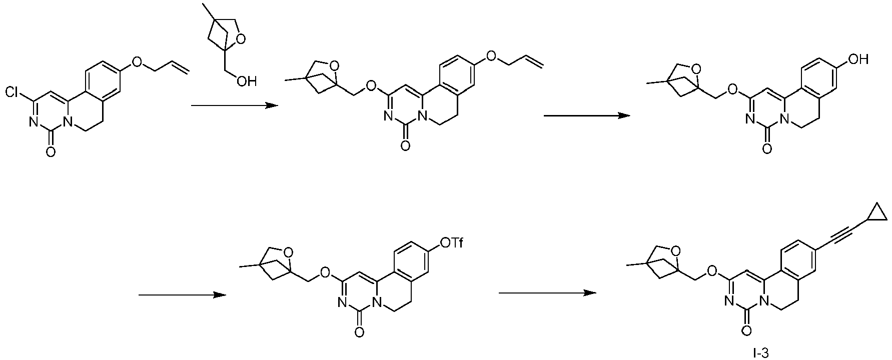

### Step 1: Synthesis of 9-(allyloxy)-2-((4-methyl-2-oxabicyclo[2.1.1]hexan-1-yl)methoxy)-6,7-dihydro-4H-pyrimido[6,1-α]isoquinolin-4-one

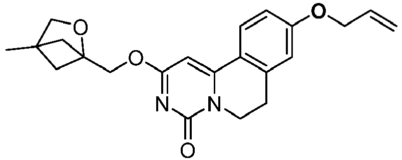

(4-Methyl-2-oxabicyclo[2.1.1]hexan-1-yl)methanol (0.23 g, 2.1 mmol) was dissolved in a solution of anhydrous DCM (100 mL) under nitrogen atmosphere at 0°C, then sodium hydride (0.081 g, 2.1 mmol, content of 60% in mineral oil) was added thereto, and the mixture was stirred for 15 minutes. 9-(Allyloxy)-2-chloro-6,7-dihydro-4H-pyrimido[6,1-α]isoquinolin-4-one (0.5 g, 1.6 mmol) was added thereto, and the reaction mixture was warmed to room temperature and stirred overnight. Saturated NH₄Cl aqueous solution (50 mL) was added thereto to wash once. The organic layer was washed once with water (50 mL), dried over MgSO₄, and concentrated under reduced pressure to obtain the crude product, and the crude product was purified by a silica gel column to obtain the product 9-(allyloxy)-2-((4-methyl-2-oxabicyclo[2.1.1]hexan-1-yl)methoxy)-6,7-dihydro-4H-pyrimido[6,1-α]isoquinolin-4-one (0.47 g, yield of 80%).

LC-MS, M/Z (ESI): 381.2 [M+H]⁺.

### Step 2: Synthesis of 2-((4-methyl-2-oxabicyclo[2.1.1]hexan-1-yl)methoxy)-9-hydroxy-6,7-dihydro-4H-pyrimido[6,1-α]isoquinolin-4-one

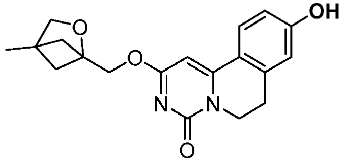

Compound 9-(allyloxy)-2-((4-methyl-2-oxabicyclo[2.1.1]hexan-1-yl)methoxy)-6,7-dihydro-4H-pyrimido[6,1-α]isoquinolin-4-one(0.45 g, 1.23 mmol) was placed in a 100 mL single-necked flask, then DCM/MeOH (10 mL/10 mL) was added thereto, and K₂CO₃ (0.34 g, 2.46 mmol) and Pd(PPh₃)₄ (0.071 g, 0.061 mmol) were added thereto at room temperature. The reaction mixture was stirred overnight at room temperature. The reaction mixture was detected by thin-layer chromatography. After the reaction was completed, the reaction solvent was concentrated to dryness to obtain the crude product, and the crude product was diluted with EA (20 mL). The organic phase was extracted three times with water (20 mL × 3), and the aqueous phases were combined, and then the pH of the aqueous phases was adjusted to 4. The aqueous phase was then extracted three times with DCM (20 mL × 3), and the organic phases were combined, washed once with saturated brine (20 mL × 1), then dried, filtered, and concentrated to obtain the crude product 2-((4-methyl-2-oxabicyclo[2.1.1]hexan-1-yl)methoxy)-9-hydroxy-6,7-dihydro-4H-pyrimido[6,1-α]isoquinolin-4-one (0.35 g, yield of 87%).

LC-MS, M/Z (ESI): 341.3 [M+H]⁺.

### Step 3: Synthesis of 2-((4-methyl-2-oxabicyclo[2.1.1]hexan-1-yl)methoxy)-4-oxo-6,7-dihydro-4H-pyrimido[6,1-α]isoquinolin-9-yl trifluoromethanesulfonate

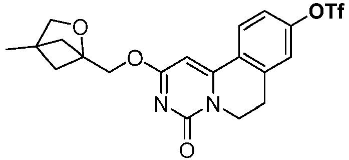

Compound 2-((4-methyl-2-oxabicyclo[2.1.1]hexan-1-yl)methoxy)-9-hydroxy-6,7-dihydro-4H-pyrimido[6,1-α]isoquinolin-4-one (0.35 g, 1.07 mmol) was dissolved in DCM (10 mL). 1,1,1-Trifluoro-N-phenyl-N-((trifluoromethyl)sulfonyl)methanesulfonamide (0.46 g, 1.28 mmol) and TEA (0.27 mL, 1.93 mmol) were added thereto, then the reaction mixture was stirred at room temperature for 5 hours, and the solvent was concentrated to dryness to obtain the crude product, which was purified by a silica gel column (DCM: MeOH (V/V) = 50:1 to 20:1) to obtain 2-((4-methyl-2-oxabicyclo[2.1.1]hexan-1-yl)methoxy)-4-oxo-6,7-dihydro-4H-pyrimido[6,1-α]isoquinolin-9-yl trifluoromethanesulfonate (0.31 g, yield of 63.4%).

LC-MS, M/Z (ESI): 473.1 [M+H]⁺.

### Step 4: Synthesis of 2-((4-methyl-2-oxabicyclo[2.1.1]hexan-1-yl)methoxy)-9-(cyclopropylethynyl)-6,7-dihydro-4H-pyrimido[6,1-α]isoquinolin-4-one

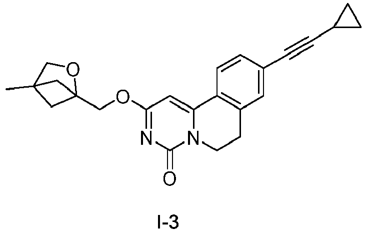

Pd(PPh₃)₂Cl₂ (0.011 g, 0.016 mmol), CuI (0.012 g, 0.065 mmol), and 2-((4-methyl-2-oxabicyclo[2.1.1]hexan-1-yl)methoxy)-4-oxo-6,7-dihydro-4H-pyrimido[6,1-α]isoquinolin-9-yl trifluoromethanesulfonate (0.15 g, 0.33 mmol) were placed in a sealed tube under nitrogen atmosphere. Cyclopropylacetylene (0.11 g, 1.64 mmol) and triethylamine (0.17 g, 1.64 mmol) were dissolved in anhydrous DMF (3 mL), added to the sealed tube and stirred overnight at 60°C. The reaction mixture was detected by thin-layer chromatography. After the raw materials were completely reacted, the reaction mixture was concentrated to remove DMF to obtain the crude product, which was purified by a silica gel column (dichloromethane: methanol (V/V) = 50:1 to 10:1) to obtain 2-((4-methyl-2-oxabicyclo[2.1.1]hexan-1-yl)methoxy)-9-(cyclopropylethynyl)-6,7-dihydro-4H-pyrimido[6,1-α]isoquinolin-4-one (I-3) (63.6 mg, yield of 51.9%).

LC-MS, M/Z (ESI): 389.2 [M+H]⁺.

¹H NMR (400 MHz, CDCl₃) δ 7.58 (d, 1H), 7.34 (dd, 1H), 7.28 (d, 1H), 6.37 (s, 1H), 4.69 (s, 2H), 4.24 - 4.11 (m, 2H), 3.63 (s, 2H), 2.95 (t,2H), 1.70-1.61 (m, 4H), 1.47 (dd, 1H), 1.34 (s, 3H), 0.96 -0.88 (m, 2H), 0.87 - 0.80 (m, 2H).

In the test examples of the present disclosure, a control compound was prepared with reference to the preparation method of compound 122 in the patent WO 2013/092791 A1, and the control compound has a structure as follows:

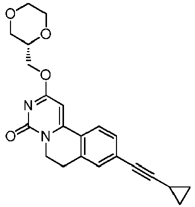

### Control compound

### Test example 1: GPR84 antagonistic effect determination experiment

The antagonistic effect of the compound against GPR84 was determined in a CHO stably transfected cell line highly expressing the human GPR84 receptor. The stably transfected cells were cultured until 80% confluence; the cells were collected by trypsinization, counted, and then inoculated into a 384-well plate at 5 µL/well. 10× compound working solution was prepared with 1× Stimulation Buffer. 1 µL of 10× compound was added to the corresponding experimental well, centrifuged, and then incubated at 37°C for 20 min; then 4 µL of 2.5 µM Forskolin & 200 nM 6-OAU solution were added thereto, centrifuged, and then incubated at 37°C for 30 min. After the reaction was completed, the content of cAMP in the cells was quantified according to the method in the instructions of the cAMP assay kit (Perkin Elmer, Cat#TRF0263). The antagonistic effect (IC₅₀ value) of the test compounds was calculated.

**Table 1 Antagonistic effect of test compounds against GPR84**

| Test compound | IC₅₀ (nM) |
|---|---|
| Control compound | 76.46 |
| I-1 | 27.23 |

The results show that the compound of the present disclosure has strong antagonistic effect against GPR84.

### Test example 2: Pharmacokinetic experiment in mice

For the pharmacokinetic experiment in mice, male ICR mice, 20 to 25 g, were used and fasted overnight. Three mice were taken and orally administered by gavage (3 mg/kg); blood was collected before the administration, and at 15 min, 30 min, 1 hour, 2 hours, 4 hours, 8 hours, and 24 hours after the administration. The blood samples were centrifuged at 6800 g for 6 min at 2 to 8°C, and plasma was collected and stored at -80°C. 20 µL of plasma at each time point was taken, and added with 200 µL of methanol containing 100 ng/mL internal standard. The mixture was vortexed, mixed evenly, and then centrifuged at 18000 g for 7 min at 2 to 8°C. 200 µL of the mixture was transferred to a 96-well injection plate for LC-MS/MS quantitative analysis. The main pharmacokinetic parameters were analyzed using WinNonlin 7.0 software with non-compartmental model.

**Table 2 Results of pharmacokinetic tests in mice**

| Test compound | Cₘₐₓ (ng/mL) | Tₘₐₓ (hr) | AUC₀₋ₜ (h * ng/mL) | T_{1/2} (h) |
|---|---|---|---|---|
| Control compound | 4119.83 | 0.50 | 7283.23 | 3.81 |
| I-1 (3 mg/kg) | 14761 | 0.50 | 54389 | 1.03 |

The results show that the compound of the present disclosure has higher oral exposure in mice and good druggability.

### Test example 3: Pharmacokinetic experiment in dogs

For the pharmacokinetic test in dogs, 3 male Beagle dogs, 8 to 10 kg, were fasted overnight and orally administered 3 mg/kg by gavage. Blood was collected before the administration, and at 15 min, 30 min, 1 hour, 2 hours, 4 hours, 8 hours, and 24 hours after the administration; the blood samples were centrifuged at 6800 g for 6 min at 2 to 8°C, and plasma was collected and stored at -80°C. The plasma at each time point was taken and added with an acetonitrile solution containing internal standard in 3 to 5 times the amount. The mixture was vortexed and mixed for 1 min, and centrifuged at 13000 rpm at 4°C for 10 min. The supernatant was collected, added with water in 3 times the amount, and mixed. An appropriate amount of the mixture was taken for LC-MS/MS analysis. The main pharmacokinetic parameters were analyzed using WinNonlin 7.0 software with non-compartmental model.

Experimental results show that the compound of the present disclosure exhibits excellent pharmacokinetic properties in dogs.

### Test example 4: Pharmacokinetic experiment in rats

The pharmacokinetic properties of the control compound and the compound of the present disclosure in rats were determined according to the following experimental methods.

Three male SD rats were used at a dose of 2.5 mg/kg, the route of administration was by gavage, and the vehicle was 5% DMSO + 10% Solutol + 85% Saline. The rats were fasted overnight, and the time points of blood collection were before administration and at 15 min, 30 min, 1 hour, 2 hours, 4 hours, 8 hours, and 24 hours after administration. The blood samples were centrifuged at 6800 g for 6 min at 2 to 8°C and plasma was collected and stored at -80°C. 20 µL of plasma at each time point was taken, and added with 200 µL of methanol containing 100 ng/mL internal standard. The mixture was vortexed, mixed evenly, and then centrifuged at 18000 g for 7 min at 2 to 8°C. 200 µL of the mixture was transferred to a 96-well injection plate for LC-MS/MS quantitative analysis. The main pharmacokinetic parameters were analyzed using WinNonlin 7.0 software with non-compartmental model.

Experimental results show that the compound of the present disclosure exhibits excellent pharmacokinetic properties in rats.

### Test example 5: Plasma protein binding rate of compounds

The plasma protein binding rate of compounds was detected by equilibrium dialysis (HTDialysis, HTD 96b). The compound was prepared into a 0.5 µM stock solution with DMSO, and then 25-fold diluted with 0.05 M sodium phosphate buffer as a working solution. A blank 96-well plate was taken and preloaded with 380 µL of plasma per well. The plasma was then added with the working solution at 20 µL/well and mixed well, with the compound at a final concentration of 1 µM, containing 0.2% DMSO per well.

100 µL of 0.05 M sodium phosphate buffer was added to the reception-side of each dialysis chamber (HTD 96b), and then 100 µL of plasma containing the compound was added to the supply-side. The dialysis chamber was covered with a plastic lid, shaken, and incubated at 37°C for 5 hours.

After incubation, 25 µL of samples were taken from each of the supply-side and the reception-side of the dialysis chamber, and placed in the blank 96-well plate. An equal volume of plasma was added to each of the supply-side samples while an equal volume of 0.05 M sodium phosphate buffer was added to each of the reception-side samples, and the mixture in each well was mixed well. The 96-well plate was added with acetonitrile solution containing internal standard at 200 µL per well, vortexed and shaken at 600 rpm for 10 min, and centrifuged at 5594 g for 15 min (Thermo Multifuge × 3R). 50 µL of the supernatant was then transferred to a new 96-well plate, and the samples were mixed with 50 µL of ultra-pure water for LC-MS/MS analysis.

The plasma protein binding rate and free fraction were calculated using the following formulas: % binding rate = 100 × ([supply-side concentration]₅ₕ - [reception-side concentration]₅ₕ) / [supply-side concentration]₅ₕ. % free fraction = 100 - % binding rate

Experimental results show that the compound of the present disclosure has a higher free fraction in human plasma and has good druggability.

### Test example 6: Stability of human liver microsomes

The stability of human liver microsomes of the control compound and the compound of the present disclosure was determined according to the following experimental method. The liver microsome stability test of the compound was detected by incubating the compound and human liver microsomes *in vitro.* First, the compound to be tested was prepared into a 10 mM stock solution in DMSO solvent, and then the compound was diluted to 0.5 mM with acetonitrile. Liver microsomes (Corning) were diluted with PBS into a microsome/buffer solution, and the solution was used to dilute 0.5 mM compound into a working solution, in which the concentration of the compound was 1.5 µM, and the concentration of liver microsomes was 0.75 mg/mL. A deep-well plate was taken, 30 µL of the working solution was added per well, and then 15 µL of pre-heated 6 mM NADPH solution was added thereto to initiate a reaction, and the reaction was incubated at 37°C. At 0, 5, 15, 30, and 45 min of the incubation, 135 µL of acetonitrile was added to the corresponding wells to terminate the reaction. After the reaction was terminated with acetonitrile at the last time point of 45 min, the deep-well plate was vortexed and shaken for 10 min (600 rpm), and then centrifuged for 15 min. After centrifugation, the supernatant was collected, and added with purified water in a ratio of 1:1 to perform LC-MS/MS detection. A ratio of a peak area of compound to a peak area of internal standard at each time point was obtained, and the peak area ratios of the compound at 5, 15, 30, and 45 min were compared with the peak area ratio at 0 min to calculate the remaining percentage of the compound at each time point. T_{1/2} was calculated by using Graphpad 5 software.

Experimental results show that the compound of the present disclosure exhibits good stability in human liver microsomes, slow metabolism, and good druggability.

Although the examples of the present disclosure are illustrated and described above, it can be understood that the above examples are illustrative and should not be construed as limiting the present disclosure. Those skilled in the art can make changes, modifications, substitutions, and variations based on the above examples within the scope of the present disclosure.

## Claims

1. A tricyclic compound, a tautomer, a stereoisomer, a hydrate, a solvate, a pharmaceutically acceptable salt, or a prodrug thereof, and the tricyclic compound has a structure of formula I: 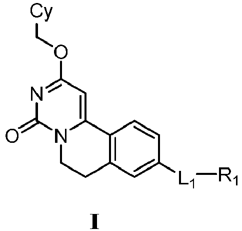 wherein
ring Cy is 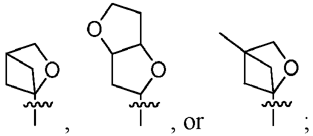
L₁ is absent or L₁ is C₁-C₄ alkylene, C₂-C₄ alkenylene with one double bond, or C₂-C₄ alkynylene with one triple bond;
R₁ is C₁-C₆ alkyl, C₁-C₆ alkoxy, 3- to 6-membered cycloalkyl, or 4- to 6-membered heterocycloalkyl;
the R₁ is optionally substituted by R₁₁;
the R₁₁ is a substituent selected from the following: halogen, cyano, hydroxyl, C₁-C₆ alkyl, C₁-C₆ alkoxy, C₃-C₆ cycloalkyl, C₁-C₆ haloalkyl, and C₁-C₆ haloalkoxy;
when there is more than one substituent, R₁₁ groups are the same or different substituents.

2. The tricyclic compound, the tautomer, the stereoisomer, the hydrate, the solvate, the pharmaceutically acceptable salt, or the prodrug thereof according to claim 1, wherein L₁ is absent or L₁ is -CH₂-, -CH=CH-, or -C≡C-;
preferably, L₁ is absent or L₁ is -C≡C-.

3. The tricyclic compound, the tautomer, the stereoisomer, the hydrate, the solvate, the pharmaceutically acceptable salt, or the prodrug thereof according to claim 1, wherein R₁ is 3-to 6-membered cycloalkyl, preferably R₁ is cyclopropyl, cyclobutyl, cyclopentyl, and cyclohexyl;
more preferably, R₁ is

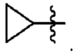

4. The tricyclic compound, the tautomer, the stereoisomer, the hydrate, the solvate, the pharmaceutically acceptable salt, or the prodrug thereof according to claim 1, wherein R₁₁ is a substituent selected from the following: halogen, cyano, C₁-C₆ alkyl, and C₁-C₆ alkoxy.

5. The tricyclic compound, the tautomer, the stereoisomer, the hydrate, the solvate, the pharmaceutically acceptable salt, or the prodrug thereof according to claim 1, wherein the tricyclic compound is:

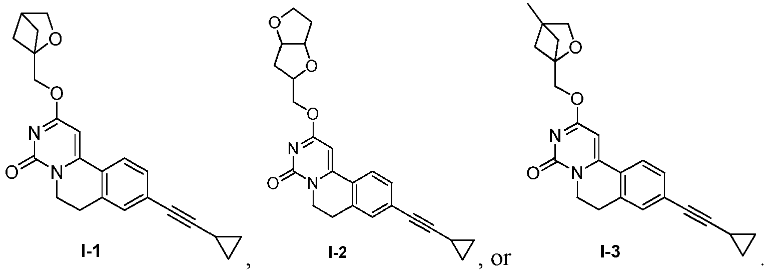

6. An intermediate B having a structure of 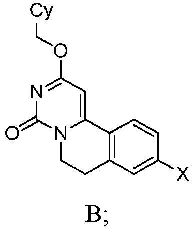
wherein Cy is as defined in claim 1;
X is selected from: -OTf, -OTs, -OMs, chlorine, bromine, or iodine.

7. A preparation method for the tricyclic compound, the tautomer, the stereoisomer, the hydrate, the solvate, the pharmaceutically acceptable salt, or the prodrug thereof according to any one of claims 1 to 5, comprising the following step: 1) reacting the intermediate B according to claim 6 with compound H-L₁-R₁ to obtain the tricyclic compound;
wherein L₁ and R₁ are as defined in claim 1.

8. The method according to claim 7, further comprising:
2) reacting the intermediate B with compound H-L₁-R₁ in the presence of a catalyst; and/or
3) reacting the intermediate B with compound H-L₁-R₁ under the protection of an inert gas; and/or
4) reacting the intermediate B with compound H-L₁-R₁ under an alkaline condition;
preferably, the catalyst is a palladium catalyst and/or a copper catalyst;
preferably, the palladium catalyst is selected from: Pd(PPh₃)₂Cl₂, Pd(OAc)₂, Pd(TFA)₂, PdCl₂, Pd(PPh₃)₄, and Pd₂(dba)₃; more preferably, the palladium catalyst is Pd(PPh₃)₂Cl₂ or Pd(OAc)₂;
preferably, the copper catalyst is a monovalent copper catalyst; more preferably, the copper catalyst is CuI;
preferably, the inert gas is nitrogen, helium, neon, or argon.

9. A pharmaceutical composition, comprising: the tricyclic compound, the tautomer, the stereoisomer, the hydrate, the solvate, the pharmaceutically acceptable salt, or the prodrug thereof according to any one of claims 1 to 5; and a pharmaceutically acceptable carrier.

10. A use of the tricyclic compound, the tautomer, the stereoisomer, the hydrate, the solvate, the pharmaceutically acceptable salt, or the prodrug thereof according to any one of claims 1 to 5, or a use of the pharmaceutical composition according to claim 9, comprising:
acting as a GPR84 antagonist;
and/or, preventing and/or treating a disease related to GPR84;
and/or, manufacturing a medicament, a pharmaceutical composition, or a preparation for acting as a GPR84 antagonist, and/or for preventing and/or treating a disease related to GPR84.

11. The use according to claim 10, wherein the disease related to GPR84 is selected from:
inflammatory diseases, pulmonary diseases, neuroinflammatory diseases, infectious diseases,
autoimmune diseases, endocrine, metabolic diseases, and diseases related to impaired immune function;
preferably, the inflammatory disease is inflammatory bowel disease or vasculitis;
preferably, the pulmonary disease is chronic obstructive pulmonary disease and/or pulmonary interstitial disease, and the pulmonary interstitial disease is preferably congenital pulmonary fibrosis or idiopathic pulmonary fibrosis;
preferably, the autoimmune disease is rheumatoid arthritis.

12. A method for preventing and/or treating a disease related to GPR84 in a subject in need, comprising administering to the subject an effective amount of the tricyclic compound, the tautomer, the stereoisomer, the hydrate, the solvate, the pharmaceutically acceptable salt, the prodrug thereof according to any one of claims 1 to 5, or the pharmaceutical composition according to claim 9.

13. The method according to claim 12, wherein the disease related to GPR84 is selected from: inflammatory diseases, pulmonary diseases, neuroinflammatory diseases, infectious diseases, autoimmune diseases, endocrine, metabolic diseases, and diseases related to impaired immune function;
preferably, the inflammatory disease is inflammatory bowel disease or vasculitis;
preferably, the pulmonary disease is chronic obstructive pulmonary disease and/or pulmonary interstitial disease, and the pulmonary interstitial disease is preferably congenital pulmonary fibrosis or idiopathic pulmonary fibrosis;
preferably, the autoimmune disease is rheumatoid arthritis.
